# EUROPÄISCHE PATENTANMELDUNG

(11) **EP 2 308 513 A2**
(43) Veröffentlichungstag der Anmeldung: **13.04.2011**
(21) Anmeldenummer: 10011333.1
(22) Anmeldetag: 16.11.2005
(51) Int. Cl.: A61K 47/48, A61K 49/00, C09K 11/88

(54) **Fluoreszenz-Nanopartikel**

(62) Teilanmeldung aus: 05025022.4
(71) Anmelder: Signalomics GmbH, 1110 Wien (AT)
(72) Erfinder: Laarmann, Sven, Dr., 59077 Hamm (DE); Zeller, Kathrien, 65520 Bad Camberg (DE)
(74) Vertreter: Von Renesse, Dorothea

(57) **Zusammenfassung**

Verwendung fluoreszierender Nanopartikel enthaltend einen anorganischen Kern, eine Passivierungsschicht und spezifische Liganden mit einem hydrodynamischen Durchmesser des anorganischen Kerns mit der Passivierungsschicht von nicht mehr als 15 nm, vorzugsweise von nicht mehr als 10 nm, besonders bevorzugt von nicht mehr als 5 nm zur Herstellung eines *in vivo* Diagnostikums, wobei die Nanopartikel eine Emission von weniger als 700 nm aufweisen.

## Beschreibung

Die Erfindung betrifft fluoreszente Nanopartikel mit einer besonderen Eignung als in *vivo* Diagnostikum, insbesondere als Kontrastmittel zur Diskriminierung verschiedener Gewebetypen.

Bei einer Velzahl von Erkrankungen ist eine möglichst frühe und aussagekräftige Diagnose von entscheidender Bedeutung für die Wahl sowie die Abstimmung und Ausführung der notwendigen medizinischen Maßnahmen. Dies gilt vor allem für eine Vielzahl von Tumorarten, für deren Bestimmung und Therapie (einschließlich möglicher Sektionen) die Diskriminierung zwischen gesundem und carcinogenem Gewebe wesentlich ist. Demnach hängt die Genesung oder sogar das Überleben eines Patienten entscheidend davon ab, ob und in welcher Güte der behandelnde und/oder operierende Arzt zwischen verschiedenen Gewebetypen unterscheiden kann.

Zur Verbesserung der Diagnose und der medizinischen Maßnahmen wurden in der Vergangenheit bereits Kontrastmittel entwickelt, mit deren Hilfe Funktionen und Strukturen im Körper über bildgebende Verfahren sichtbar gemacht werden können. Diese Verfahren werden unter anderem zur gezielten Detektion krebsassoziierter Zellveränderungen verwendet.

So haben beispielsweise Hsu et al. (2004) ("A far-red fluorescent contrast agent to image epidermal growth factor receptor expression", Photochemistry and Photobiology, 79 (3): 272-279) ein molekular spezifisches Kontrastmittel auf der Basis eines organischen Fluorophors als Marker für die frühe carcinogene Transformation entwickelt. Hierin wird die tumorassoziierte Überexpression des epidermalen Wachstumsfaktor-Rezeptors (EGFR, epidermal growth factor receptor) ausgenutzt, um verändertes Gewebe in der Mundhöhle über ein rot fluoreszierendes anti-EGFR-Antikörper Konjugat (Alexa660) zu identifizieren.

Generell ist ein Nachteil organischer Fluorophore deren Metabolisierung im Körper, bei der das Fluorochrom abgebaut oder inaktiviert wird. Die Metabolisierung wirkt damit der für die Diagnostik notwendigen hohen Markierungsintensität entgegen. Mit zunehmender Verweildauer des organischen Fluorophors *in vivo* verschärft sich diese Problematik und stellt insbesondere bei der Markierung von Zellen in tieferen Gewebeschichten eine erhebliche Schwierigkeit dar.

Des Weiteren haben insbesondere längerwellig emittierende organische Fluorophore den Nachteil, dass sich deren Quantenausbeute durch den chemischen Konjugationsprozess reduziert. Zudem sind organische Fluorophore sehr anfällig gegenüber Photobleichung ("photo bleaching"), was schon nach kurzer Bestrahlung zu einem substantiellen Fluoreszenzverlust führen kann. Dadurch weist ein Kontrastmittel auf der Basis dieser Fluorophore eine zu geringe Fluoreszenzstärke und Stabilität bei längerer Anregungsdauer auf, um für die Detektion/Markierung von Zellen in tieferen Gewebeschichten geeignet zu sein ("deep tissue imaging"). So geht aus der Studie von Hsu et al. (2004) hervor, dass die Alexa660-Konjugate eine Eindringtiefe von maximal 0,5 mm aufweisen, so dass eine Detektion der Fluoreszenz nicht mehr sinnvoll möglich ist.

Eine weitere bekannte Möglichkeit zur Fluoreszenzmarkierung zellulärer Veränderungen besteht in der Verwendung von sog. Quantum Dots (QDs), bei denen es sich um wenige Nanometer große fluoreszente Nanopartikel handelt, deren Kern aus Halbleitermaterialien wie CdSe, CdTe, InP oder ähnlichem besteht.

Die bekannten QD zeigen bei ihrer Verwendung in biologischen Systemen jedoch das sogenannte "blinking"-Phärlomen, d.h. die Nanopartikel wechseln zwischen einem fluoreszenten und einem nicht-fluoreszenten Zustand. Dieses Phänomen macht die Quantum Dots insbesondere für die *in vivo* Anwendung untauglich. Zudem kann das "Blinken" auch einen Zerfall des Nanopartikelkems anzeigen, aufgrund dessen toxisches Cadmium in den Körper freigesetzt werden kann. Dies ist besonders nachteilig, da die Quantum Dots im Körper, so beispielsweise in der Leber oder in der Milz, akkumulieren.

Es liegt demnach der vorliegenden Erfindung die Aufgabe zugrunde, fluoreszente Nanopartikel bereitzustellen, die eine besondere Eignung zur Verwendung als Diagnostikum, insbesondere als Kontrastmittel in *vivo* aufweisen.

Diese Aufgabe wird gemäß dem Hauptanspruch gelöst. Vorteilhafte Ausführungsformen sind Gegenstand der Unteransprüche bzw. separater Nebenansprüche. Die erfindungsgemäßen Nanopartikel können sowohl *in vitro* als auch *in vivo* zur spezifischen Markierung ausgewählter biologischer Strukturen oder Funktionen eingesetzt werden.

Die erfindungsgemäßen Nanopartikel umfassen mindestens drei Strukturen, nämlich einen anorganischen Kern. der mit einer Passivierungsschicht ummantelt wird, die dann ihrerseits spezifische Liganden trägt, wobei die spezifischen Liganden auch Teil der Passivierungsschicht sein können. Diese führen zu der spezifischen Bindung des Nanopartikels an das Zielmolekül ("targer) des biologischen Systems. Der anorganische Kern der erfindungsgemäßen Nanopartikel mit der ihn ungebenden Passivierungsschicht weist einen hydrodynamischen Durchmesser von nicht mehr als 15, vorzugsweise nicht mehr als 10 nm auf. Besonders bevorzugt sind thermodynamische Durchmesser von nicht mehr als 8 nm oder nicht mehr als 5 nm.

Der Passivierungsschicht kommt vor allem die Aufgabe zu, die Fluoreszenzintensität sowie die chemische und physikalische Stabilität des anorganischen Kerns zu erhöhen. Die mit der Passivierungsschicht ummantelten anorganischen Kerne sind gekennzeichnet durch eine Quantenausbeute von mindestens 10 %, vorteilhafterweise mindestens 30, 50 oder sogar 70 %. Als Quantenausbeute wird dabei das Verhältnis der Menge des von einer Probe emittierten Lichts zu der Menge des von der Probe absorbierten Lichts verstanden. Die Passivierungsschicht weist vorteilhafterweise eine Dicke von nicht mehr als 1 nm auf. Der Durchmesser des passivierten Kerns erhöht sich in diesem Fall um nicht mehr als 2 nm.

Vorteilhafterweise werden die Nanopartikel jeweils noch mit Modifikatoren, insbesondere zur Verbesserung der Kompatibilität mit der biologischen Umgebung versehen. Die Zunahme des hydrodynamischen Radius durch die Verwendung von Modifikatoren überschreitet vorzugsweise 2 nm nicht. Die Dicke der Passivierungsschicht und der Modifikatoren hängt im Einzelfall auch von den Verhältnissen belder Strukturen untereinander und im Verhältnis auf den anorganischen Kern ab.

Aufgrund der Größenbeschränkung der erfindungsgemäßen Nanopartikel weisen sie eine besondere Eignung zur Verwendung als Diagnostikum im lebenden Patienten auf. So wird durch die Größenreduktion eine Erhöhung der Diffusionsgeschwindigkeit und der Eindringtiefe in das Gewebe erreicht. Dies erlaubt eine gleichmäßige und schnelle Ausbreitung der Nanopartikel in der biologischen Umgebung sowie eine möglichst weitgehende Durchdringung eines Gewebes (z.B. eines Tumors) nach einer lokalen Applikation. Ebenso erlauben die erfindungsgemäßen Nanopartikel eine systemische Applikation, die auch über eine Injektion erfolgen kann. Auch ist eine lokale, z.B. eine topische Applikation möglich.

Besonders vorteilhafte Ausführungsformen der erfindungsgemäßen Nanopartikel weisen einen hydrodynamischen Durchmesser von nicht mehr als 8, besonders bevorzugt von nicht mehr als 4 nm auf. Nanopartikel dieser Größenordnung können bereits über die Niere ausgeschieden werden, so dass sie nicht oder deutlich weniger im Körper akkumulieren. Somit vermindern die erfindungsgemäßen Nanopartikel das mit den bekannten Quantum Dots voraussichtlich verbundene Problem der Langzeittoxizität erheblich.

In einer weiteren vorteilhaften Ausführungsform emittieren die erfindungsgemäßen Nanopartikel ein Fluoreszenzspektrum zwischen 600 und 700 nm, besonders bevorzugt von 600 bis 650 nm, insbesondere bevorzugt 620 bis 650 nm. Dieses Emmissionspektrum hat den Vorteil der möglichst hohen Gewebetransmission aufgrund einer nur geringen Absorption durch Hämoglobin und anderer lichtabsorbierende Substanzen im lebenden System (einschließlich Wasser). Licht dieser Wellenlängen ist für das menschliche Auge noch wahrnehmbar, so dass die Identifizierung des markierten Gewebes durch den behandelnden Arzt ohne weitere aufwändige technische Hilfsmittel zur Detektion (z.B. CCD-Kameras) möglich ist. Dies ist insbesondere bei der Verwendung der erfindungsgemäßen Nanopartikel als Kontrastmittel während eines chirurgischen Eingriffs zur Diskriminierung des (beispielsweise) carcinogenen und gesunden Gewebes vorteilhaft.

Es ist von besonderem Vorteil, wenn der anorganische Kern der erfindungsgemäßen Nanopartikel im wesentlichen aus Halbleitern besteht. Diese Kerne emittieren je nach ihrer individuellen Größe und/oder Zusammensetzung Licht in verschiedenen Farben, absorbieren aber alle breitbandig im selben Bereich des Lichtspektrums (UV bis VIS Bereich). Die Anregungs- und Emissionsspektren liegen aufgrund des hohen "Stokes shift" weit auseinander, was eine einfache und gleichzeitige Anregung verschiedener Quantum Dots ermöglicht. Sie weisen schmale und symmetrische Emissionsspektren auf, die sich nicht bzw. nur geringfügig überlappen. Weitere positive Eigenschaften, die insbesondere für die verbesserte Eindringtiefe und die *in* vivo Markierung von großer Bedeutung sind, stellen die hohe Quantenausbeute von bis zu 80% und die hohe Photostabilität dar.

Quantum Dots, die den anorganischen Kern der erfindungsgemäßen Nanopartikel darstellen können, sind aus der WO2005/001889 bekannt. Danach handelt es sich um einen anorganischen Kern aus einer Legierung aus mindestens zwei Halbleitern, die entweder homogen verteilt sind oder aber für die innerhalb der Legierung jeweils ein Konzentrationsgradient vorliegt. Hinsichtlich der Offenbarung der Beschaffenheit und der Herstellung dieser Quantum Dots wird auf oben zitierte WO2005/001889 verwiesen. Die Kerne können in ihrer Größe um jeweils 5 % abweichen.

Demnach kann der anorganische Kern der erfindungsgemäßen Nanopartikel eine Legierung aus mindestens zwei Halbleitern umfassen, wobei der Kern eine homogene Zusammensetzung aufweist und durch eine "Band-Gap-Energie" charakterisiert ist, die nicht-linear zu dem molaren Verhältnis der zwei Halbleiter ist. Alternativ kann der Kern nicht-homogen beschaffen sein, wobei die Konzentration des ersten Halbleiters graduell ansteigt ausgehend vom Zentrum des Kerns bis zur Oberfläche des Kerns und die Konzentration des zweiten Halbleiters graduell abnimmt vom Zentrum des Kerns bis zu dessen Oberfläche.

Es gilt für beide Kerne gleichermaßen, dass mindestens einer der Halbleiter ein Gruppe 11-Gruppe VI-Halbleiter oder ein Gruppe 111-Gruppe V-Halbleiter ist (die Gruppendefinition korrespondiert mit den Gruppen des Periodensystems der Elemente). Die Legierung kann beispielsweise ausgewählt sein aus der Gruppe folgender Legierungen: CdSeTe, CdSSe, CdSTe, ZnSeTe, ZnCdTe, CdHgS, CdHgTe, InGaAs, InGaP, GaALAs, InGaN. Diese Kerne können zudem eine Beschichtung aus anorganischem Material wie z.B. Halbleitern (z.B. ZnS) tragen. Diese zusätzliche Schicht ist dem Fachmann als "capping" oder "shell" bekannt.

Gruppe II-Gruppe VI und Gruppe III-Gruppe V Halbleiter sind allgemein bekannt und beinhalten z.B. CdS₁₋ₓSeₓ, CdS₁-ₓTeₓ. CdSe₁₋ₓTeₓ, ZnSe₁₋ₓTeₓ₁ Zn₁₋ₓCdₓTe, Cd₁. ₓHgₓS, Cd₁₋ₓHgₓTe, In₁₋ₓGaₓAs, Ga₁₋ₓAlₓAs und In₁₋ₓGaₓP. Vorzugsweise werden die Halbleiter CdSe₁₋ₓTeₓ, CdS₁₋ₓTeₓ, ZnSe₁₋ₓTeₓ. Zn₁₋ₓCdₓTe, Cd₁₋ₓHgₓS, Cd₁₋ₓHgₓTe, In₁₋ₓGaₓAs, In₁₋ₓGaₓP verwendet, wobei x eine Fraktion von 0 bis 1 ist.

Das molare Verhältnis der Halbleiter kann jedes molare Verhältnis annehmen. Allerdings ist in dem Fall, dass die Legierung CdSSe umfasst, eine Legierung mit der molekularen Formulierung CdS₁₋ₓSeₓ bevorzugt. In dem Fall, dass die Legierung CdSTe umfasst, ist eine Legierung mit der molekularen Formulierung CdS₁₋ₓTeₓ bevorzugt. In dem Fall, dass die Legierung ZnSeTe umfasst, ist eine Legierung mit der molekularen Formulierung ZnSe₁₋ₓTeₓ bevorzugt. In dem Fall, dass die Legierung ZnCdTe umfasst, ist eine Legierung mit der molekularen Formulierung allein aus CdTe bevorzugt. In diesen Angaben ist x jeweils eine Fraktion zwischen 0 und 1.

Diese bevorzugten anorganischen Kerne der erfindungsgemäßen Nanopartikel können mit folgenden Schritten hergestellt werden: (i) die Herstellung einer ersten Lösung unter Bedingungen, die die Bildung von Nanokristallen ermöglichen, (ii) Herstellung einer zweiten Lösung, die eine Vorstufe der Halbleiter umfasst mit einem molaren Verhältnis unter einer Bedingung, welche keine Bildung von Nanokristallen ermöglicht, (iii) Zugabe der zweiten Lösung zur ersten Lösung, die eine Bildung von Nanopartikeln ermöglicht, und (iv) Veränderung der Bedingungen, die das Wachstum der Nanokristalle und ihrer Bildung anhalten/stoppen. Das Verfahren zur Herstellung der Kerne wird in der WO 2005/001889 dargestellt, auf die hinsichtlich der Offenbarung der Herstellung dieser bevorzugten Ausführungsform des anorganischen Kerns der erfindungsgemäßen Nanopartikel Bezug genommen wird.

In einer alternativen Ausführungsform kann der anorganische Kern im Wesentlichen aus einem Edelmetallcluster bestehen, der vorzugsweise 2 und 27 Edelmetall-Atome umfasst. In einer bevorzugten Ausführungsform wurde das Edelmetall aus einer Gruppe bestehend aus Gold, Silber, Kupfer, Platin, Palladium, Osmium, Indium, Ruthenium und Rhodium ausgewählt. Das Cluster kann variierende Ladungen aufweisen.

Diese Kerne haben den Vorteil, dass sie aufgrund ihrer starken Absorption und Emission leicht als einzelne sogenannte "Nanodots" mit einer schwachen Quecksilber-Lampen-Anregung detektierbar sind. Die erfindungsgemäßen Nanopartikel mit diesen Kernen sind vorteilhaft als fluoreszentes Einzelmolekül- und Massenlabel zu verwenden.

Im Sinne der vorliegenden Erfindung bezieht sich der Begriff "Edelmetall" auf eine Elementengruppe ausgewählt aus einer Gruppe bestehend aus Gold, Silber und Kupfer und der Gruppe der Platinmetalle (PGM) Platin, Palladium, Osmium, Iridium, Ruthenium und Rhodium. In bevorzugten Ausführungsformen der vorliegenden Erfindung werden die Edelmetalle aus der Gruppe bestehend aus Gold, Silber und Kupfer ausgewählt. In einer besonders bevorzugten Ausführungsform ist das Edelmetall Silber oder Gold.

Der Begriff "Cluster" bezieht sich auf eine Verbindung von 2-27 Atomen eines Metalls. Cluster sind u. a. aus den Bereichen der chemische Katalyse, der Keramik, der Halbleitertechnologie und der Materialwissenrchaften bekannt. Ihre Herstellung ist dem Fachmann daher geläufig. Die WO2004/003558 beschreibt u.a. die Herstellung von Edelmetallclustem und enthält darüber hinaus umfangreiche weiterführende Literaturangaben dazu. Insbesondere ist die Herstellung von Edelmetall-Nanoclustem offenbart, die mit organischen Molekülen assoziiert sind. Der Begriff Assoziation ist dabei zu verstehen als jede Form der Bindung unabhängig von der chemischen oder physikallschen Natur der Bindung (so z.B. kovalente, nichtkovalente, elektrostatische oder van-der-Waals-Bindung). Hinsichtlich der Herstellung der Nanocluster als Kern der erfindungsgemäßen Nanopartikel wird auf die WO20041003558 in Bezug genommen.

Die erfindungsgemäßen Nanopartikel weisen eine Passivierungsschicht auf, die die Fluoreszenzintensität erhöht und die chemische und physikalische Stabilität des anorganischen Kerns verbessert. Damit emittieren die Nanopartikel Licht vorzugsweise mit einer Quantenausbeute von mehr als 10 %, vorzugsweise von mehr als 50 %.

Die erfindungsgemäßen Nanopartikel weisen in wässriger Umgebung bei 4 °C vorzugsweise eine Lagerstabilität von mindestens 12 Monaten auf und sind vorzugsweise über einen pH-Bereich von pH 5 bis pH 10 stabil, d.h., sie zeigen Abweichungen von weniger als 50 % in Bezug auf ihre spezifischen spektralen Charakteristika wie Quantenausbeute, Lage des Emissionsmaximums, Halbwertsbreite des Emissionsspektrums. Bevorzugte Partikel zeigen Abweichungen von weniger als 10 % in Bezug auf diese spezifischen spektralen Charakteristika.

Auch zeigen sie unter biologischen Bedingungen bzw. in *vivo* für einen Zeitraum von mindestens drei Tagen im wesentlichen eine Konstanz/Stabilität der Eigenschaften des Kerns (einschließlich der ihn umgebenden Passivierungsschicht). Bevorzugte Partikel zeigen eine derartige Konstanz/Stabilität für einen Zeitraum von 7 bis 14 Tagen bis hin zu mehreren Wochen, wobei als Konstanz im Sinne der Erfindung eine Abweichung/Veränderung einiger. oder aller der oben genannten Eigenschaften um 50 % verstanden wird. Besonders bevorzugte Partikel zeigen eine Abweichung/Veränderung kleiner als 10 %.

Die Passivierungsschicht enthält mindestens eine Verbindung, die in der Lage ist, Metallatome oder Metallionen, z.B. Zink-, Quecksilber- oder Cadmiumionen zu koordinieren. Diese Verbindung kann eine Lewis-Base oder eine cyclisch oder linear ungesättigte Verbindung mit resonanten Elektronen sein. Als cyclisch ungesättigte Verbindung kann sie auch ein Heterozyklus bzw. ein Heteroaromat sein. Die ungesättigte oder konjugierte Gruppe befindet sich in einer bevorzugten Ausführungsform in einer terminalen Position bezogen auf die Struktur des Moleküls. Weiterhin kann die Passivierungsschicht einen Quervernetzer aufweisen oder die cyclisch oder linear ungesättigte Verbindung kann auch als Quervemetzer fungieren.

Die Metallatome oder Metallionen koordinierenden Verbindungen können funktional durch Chelatisierung, Koordination oder Elektronen-Dvnor Eigenschaften von Lewis-Basen an fluoreszente anorganische Kerne binden und entsprechend konjugierte Anteile/Gruppen aufweisen. Diese Moleküle können zudem Anteile enthalten, die den Kernen, die mit ihnen beschichtet sind, in wässrigen Lösungen Löslichkeit oder Benetzbarkeit vermitteln.

Diese Moleküle oder Verbindungen können ein homogenes oder heterogenes (heterocyclisches) Ringsystem mit ein, zwei oder mehr gebundenen (oder auch kondensierten) Ringen beeinhalten, Bevorzugte heteroaromatische Systeme sind z.B. Thiazole, Thiazolderivate, Oxazole, Oxazolderivate, Pyrrole, Pyrrolderivate einschließlich dotierter oder nicht-dotierter Polypyrrol-Oligomere, Thiophene, Thiophenderivate einschließlich dotierter und nicht-dotierter Polythiophene, Furane, Furanderivate, Pyridin und ―derivate, Pyrimidin und seine Derivate, Pyrazine, Pyrazinderivate, Triazin und ―derivate. Triazole, Triazolderivate, Phthalocyanine und -derivate, Porphyrin und Porphyrinderivate. Diese Verbindungen können ungesättigte (olefinische) Kohlenwasserstoffe oder deren Amine, Phosphor- oder Sauerstoffderivate einhalten, die Acetylen, Propin und Allen mit einschließen können, aber nicht auf diese begrenzt sind. Es ist zu bevorzugen, dass das Molekül eine ausreichende p oder pi-Etektronen-Dichte aufweist, um sich an der Adduktbildung oder der Resonanz auf der Oberfläche des Halbleiter-Kerns zu beteiligen.

Die heteroaromatische Verbindung ist vorzugsweise eine Imidazol-Komponente. Vorzugsweise wird weiterhin als Quervemetzer eine Alkylphosphin-Verbindung zugesetzt.

Mit dem Begriff "Imidazol-Komponente" wird im Sinne dieser Beschreibung ein heterozyklisches oder heteroaromatisches Molekül verstanden, das mindestens eine Imidazol-Gruppe (einschließlich Imidazol-Derivate) enthält, und das für die Bindung des anorganischen Kerns oder der Passivierungsschicht mit einem Metall wie Cadmium, Zink, Gallium oder einem Metallkation oder einem Substrat, das ein solches Kation enthält, zur Verfügung steht. In diesem Zusammenhang sollte vorzugsweise mindestens eine Imidazol-Gruppe in einer terminalen Position bezogen auf die Struktur des Moleküls sein. Die Imidazol-Komponente bindet In Ihrer funktionellen Form über den Ring, der delokalisierte Molekülorbitale enthält, an den fluoreszenten Nanokristall. Gewöhnlich dienen die Stickstoffe des Imidazolrings als koordinierende Liganden, um in funktioneller Weise ein Metall-Ion wie Cadmium oder Zink zu binden.

In einer Ausführungsform umfasst die Imidazol-Komponente reaktive funktionelle Gruppen wie eine oder zwei Aminosäure(n), z.B. Histidin, Carnosin, Anserin, Balein, Homocamosin, Histidylphenylalanin, cyklo-Histidylphenylalanin, 5-amino-4-Imidazol-carboxamid, Histidylleucin, 2-Mercaptoimidazol, boc-Histidin, Hydrazid, Histinol, 1-Methylhistidin, 3-Methylhistidin, Imidazolysin, Imidazol-enthaltendes Ornithin (z. B. 5-Methylimidazol), Imidazol-enthaltendes Alanin (z. B. (beta)-(2-Imidazolyl)-L(alpha) Alanin), Carzinin, Histamin. Diese auf Histidin basierenden Moleküle oder Imidazolhaltige Aminosäuren können nach allgemein bekannten Methoden synthetisiert werden.

Unter dem Begriff "Alkylphosphin" wird im Sinne der Erfindung ein Molekül verstanden, das mindestens eine Phosphingruppe (einschließlich ihrer Derivate) für die Bindung oder das Chelatisieren eines Nichtmetalls wie Se, S oder anderer Nichtmetalle aufweist oder von Substraten, die solche Atome enthalten und das mindestens eine funktionelle Gruppe (z.B. Hydroxyl-, Amino-, Thiol-, Carboxyl-, Carboxamid- etc.) zur Reaktion mit benachbarten Molekülen bereitstellt.

Vorzugsweise sollte mindestens eine Phosphin-Gruppierung in einer terminalen Position bezogen auf die Struktur des Moleküls lokalisiert sein. Die Phosphin-Anteile dienen als koordinierende Liganden, um in funktionaler Form mit einem fluoreszenten Kern oder einer Verbindung aus der abschirmenden Schicht ein Nichtmetall oder Ion wie Se oder S zu binden.

In einer bevorzugten Ausführungsform beinhaltet die Alkylphosphin-haltige Verbindung eine, zwei oder mehrere miteinander (z.B. in polymerer Form) gekoppelte Phosphin-Gruppen, die Hydroxymethylphosphin-Verbindungen oder ähnliches mit einschließen können, aber nicht auf diese begrenzt sind. Alkylphosphin-enthaltende Verbindungen können nach allgemein bekannten Methoden synthetisiert werden. Wie weiterhin bekannt ist, können Alkylphosphin-haltige Verbindungen außerdem eine oder mehrere zusätzliche funktionelle Gruppen (z.B. Hydroxyl-, Amino-, Thiol., Carboxyl-, Carboxamid. etc.) aufweisen. Beispiele für Derivate sind Hydroxymethlyphosphinderivate, Amide oder Ester, sofern die Derivatisierung mit den hier beschriebenen Funktionen des Alkylphosphins als Coating vereinbar ist.

Besonders bevorzugt sind Tris-(hydroxymethyl-)phosphin und β-[Tris-(hydroxymethyl)phosphino-)propansäure, um die fluoreszenten anorganischen Kerne der erfindungsgemäßen Nanopartikel zu beschichten. Allgemein bekannt ist, dass quervernetzte Alkylphosphin-hakige Verbindungen zusätzlich die Möglichkeit haben, funktional an Metallatom und/oder -ionen wie Zn oder Cd zu binden. In dieser Hinsicht funktionalisierte Isocyanate oder Alkylcyanoacrylate können weiterhin als Quervemetzer für Liganden und Adduktbildung mit fluoreszenten Kernen nützlich sein.

Dem passivierenden Effekt der erfindungsgemäß vorhandenen Passivierungsschicht liegt das Abschirmen von oberflächlichen Cadmium- oder Zinkatomen oder ähnlichem durch die Komplexierung mit dem Heteroaromaten oder Heterozyklus (vorzugsweise mit der Imidazol-Komonente) und das Abschirmen der Gegenatome (Se oder S oder Ähnliches) über die Komplexierung mit den Alkylphosphin-haltigen Verbindungen zugrunde.

Die Passivierungsschicht der erfindungsgemäßen Nanopartikel ist aus der US 2004/0247861 A1 bekannt. In dieser Offenlegungsschrift wird die Herstellung von mit der Passivierungsschicht ummantelten anorganischen Kernen, zum Beispiel von Quantum Dots beschrieben. Zu Zwecken der Offenbarung der Hersteltung der erfindungsgemäß eingesetzte Passivierungsschicht und der damit ummantelten anorganischen Kerne wird die US 200410247861 daher in Bezug genommen.

Die Moleküle der Passivierungsschicht können weiterhin chemische Gruppen aufweisen oder tragen, um Zielmoleküle und Zellen zu binden und querzuvernetzen (spezifische Liganden). In der Gegenwart entsprechend passender Reagenzien wie ZnSO₄ und Na₂S können diese Moleküle oder Verbindungen mit den Molekülen auf dem fluoreszenten Kern eine Passivierungsschicht bilden ("capping" oder "shell"). Diese Reagentien können auch an Atome oder Ionen auf der Oberfläche des fluoreszenten Nanokristalls funktional binden, so dass diese zusätzliche Passivierungsschicht auch unmittelbar auf der Oberfläche des Kerns ausgebildet sein kann.

Die erfindungsgemäßen Nanopartikel können in einer vorteilhaften Ausführungsform zusätzlich Modifikatoren aufweisen, die aus organischen und/oder anorganischen Anteilen bestehen können. Sie dienen der verbesserten Kompatibilität, Effektivität und/oder Löslichkeit der Nanopartikel in einer Flüssigkeit oder einem Suspensionsmedium insbesondere in der physiologischen Umgebung. Diese Oberflächenmodifikation ist vor allem vorteilhaft, um eine möglichst geringe unspezifische Adsorption und eine erhöhte Verträglichkeit in biologischen Systemen, insbesondere im menschlichen Körper, zu erreichen.

Eine Möglichkeit ist die Modifikation der Oberfläche mit dem für bestimmte medizinische Anwendungen bereits zugelassenen Polyethylenglykol' (PEG), insbesondere in niedermolekularen Formen, um eine geringe Gesamtgröße des Nanopartikels beizubehalten. Dies kann sowohl die Biokompatibilität der Nanopartikel sowie deren Blutzirkulativnszeit und auch die Aufnahmeeffizienz in Zellen erhöhen. Durch die Kombination einer niedermolekularen PEG-Schicht mit weiteren Substanzen wie Vitaminen wie z.B. Folsäure, kann eine geringere Aufnahme der Nanopartikel in Makrophagen erzielt werden, da aufgrund der damit reduzierten Proteinadsorption an den Nanopartikeln eine Erkennung der Nanopartikel durch das Immunsystem erschwert wird.

Die Beschichtung mit Monosacchariden, Di- oder Trisacchariden bis hin zu niedermolekularen Polysacchariden aus einem oder verschiedenen Monosacchariden stellt eine weitere Möglichkeit der vorteilhaften Oberflächenmodifikation durch Verwendung von Modifikatoren dar. Als eine Ausführungsart ist eine Modifikation mit z.B. Polyglucose möglich, in der Dextran eingesetzt werden kann, welches als Blutersatzstoff medizinisch zugelassen ist. Es zeigt eine gute Biokompatibilität/Verträglichkeit. Eine weitere Ausführungsform ist die Verwendung von stereoisomeren Formen (D-/L-) der Saccharide, um einer möglichen Degradation entgegenzuwirken.

Eine weitere Ausführungsform ist die Verwendung von biologisch kompatiblen hydrophilen Vitaminen als Modifikatoren wie z.B. Thiamin. Riboflavin, Niacin, Pyridoxin, Cobalamin, Panthothensäure, Ascorbinsäure und Folsäure. So kann z.B. Folsäure zu einer bevorzugten Bindung von Nanopartikeln an Krebszellen führen. Dieses Vitamin zeigt nur eine geringe Immunogenität und somit eine hohe Biokompatibilität. Durch die Bindung an den membranständigen Folsäurerezeptor wird eine Internalisierung der Nanopartikel erleichtert.

Die Oberflächenmodifikation mit lipophilen Vitaminen wie Retinol, Cholecalciferol, Tocopherol und Phyllochinon ist ebenso möglich. So kann z.B. Vitamin E zu einer erhöhten zellulären Aufnahme von Nanopartikeln führen.

Fettsäuren, wie z.B. 1-Oktadecene oder 18-Methyleicesanoidsäure und deren Derivate, können die Löslichkeit und Stabilität der Kolloide erhöhen und verfügen über eine terminale funktionelle Carboxylgruppe, die zur nachfolgenden Bindung spezifischer Liganden genutzt werden kann. Daher ist es sinnvoll, Fettsäuren als Modlflkatoren mit aufzunehmen.

Eine weitere Ausführungsform der Oberflächenmodifikation ist eine Beschichtung mit Polyalkoholen, wie z.B. Diethylenglykol (DEG), die besonders gut unspezifische Proteinadsorption reduzieren können. Gleiches gilt für Polytetrafluoroethylen (PTFE, Teflon), insbesondere in seinen niedermolekularen Formen, aufgrund derer eine reduzierte Proteinadsorption erreicht werden kann. Polytetrafluoroethylen wird häufig in kardiochirurgischen Applikationen eingesetzt.

Eine Oberflächenmodifikation kann ebenfalls mit einer oder mehreren natürlich vorkommenden Aminosäuren, zu denen sowohl die proteinogenen als auch nichtproteinogenen Aminosäuren zählen, sowie synthetischen Aminosäuren vorgenommen werden. Dabei können beide Stereoisomere (D- und L-Formen) verwendet werden. Di-, Tri-, Tetra- bis hin zu kleinen Polypeptiden aus den oben genannten Aminosäuren stimulieren kaum das Immunsystem und sind somit ebenfalls für eine dünne Kompatibilitätsschicht geeignet. Dabei kann es sich um künstliche Aminosäuresequenzen als auch Sequenzen aus biologischen Proteinen handeln. Peptidabkömmlinge von natürlichen Proteinen wie z.B. des Phytochelatins können ebenfalls zur Oberflächenmodifikation verwendet werden. Eine Oberflächenmodifikation mit Tat-Peptid und Tat-Peptid enthaltenden Peptiden ist eine weitere Möglichkeit, Nanopartikel für den Einsatz In blomedizinischen Applikationen verfügbar zu machen. Das Tat-Peptid ist ein effektives Molekül, um z.B. Goldnenopartikel durch die Zellmembran bis in den Kern zu bringen.

Eine weitere Ausführungsform der möglichen Modifikatoren ist die Ausbildung einer Phosphorylcholin-Beschichtung. Phosphorylcholin reduziert eine mögliche unspezifische Proteinadsorption, wie z.B. auf Kontaktlinsen. Eine Phophorylcholin-Modifikation kann aufgrund der nicht-thrombogenen Eigenschaften gut in biologischen Systemen eingesetzt werden und zeichnet sich durch eine hohe Langzeitstabilität aus.

Da Polylactat biokompatibel ist, wird diese Substanz in vielfältigen medizinischen Anwendungen eingesetzt. Insbesondere niedermolekulare Formen des Polylactats sind eine weitere Möglichkeit der Oberflächenmodifikation der erfindungsgemäßen Nanopartikel. Dabei können beide Stereoisomere (D-/L-Form) eingesetzt werden, um eine mögliche Biodegradation zu reduzieren.

Neben den genannten Oberflächenmodifikationen ist eine proteolytisch spaltbare Bindung von unspezifischen Proteinen an die Nanopartikel möglich. Dies kann eine Erhöhung der Biokompatibiiität/Vertragtichkeit bewirken. Am Zielort kann eine Abspaltung des großen Proteins erfolgen unter Freisetzung der kleinen Nanopartikel im Gewebe. Ebenso kann die Abspaltung nach entsprechender Verweildauer erfolgen. Dazu eignen sich bevorzugt weit verbreitete Proteine wie z.B. Transferrin, Lactoferrin, Ceruloplasmin, Elastin und Albumin neben anderen Proteinen, die eine unspezifische Adsorption reduzieren. So kann z.B. eine Oberflächenbeschichtung aus Kombinationen von Polypeptiden mit Elastin unerwünschte Thrombenbildung verhindern und somit die Biokompatibilität der Nanopartikel erhöhen.

Das Hauptserumprotein Albumin kann nichtspezifische Interaktionen mit Plasmamembranen reduzieren. Desweiteren behält das entsprechend modifizierte Nanopartikel die Fähigkeit, spezifische Interaktionen mit Zielzellen durch gleichzeitige Bindung eines spezifischen Liganden an der Partikeloberfläche auszubilden. Eine Beschichtung mit Serumalbumin kann zu einer wesentlich längeren Blutzirkulationszeit durch die Verhinderung einer schnellen Aufnahme durch Makrophagen nach intravenöser Gabe führen als dies bei unbeschichteten Nanopartikeln der Fall ist.

Neben den oben skizzierten unspezifischen Beschichtungen tragen die erfindungsgemäßen Nanopartikel eine selektive Markierung mit Zielzell-spezifischen Liganden, beispielsweise sind sie konjugiert mit Proteinen, Antikörpern. Peptiden oder, besonders bevorzugt, mit kleinen, hochaffinen Proteindomänen, Antikörperfragmenten oder anderen organischen Molekülen, die z.B. an Tumorzell-spezifische Strukturen oder andere Targets binden.

Die Kombination aus reduziertem hydrodynamischen Durchmesser, der zu der erwähnten höheren Diffusions- und Perfusionsgeschwindigkeit führt, zusammen mit den bereits beschriebenen Eigenschaften und Verbesserungen und der hohen Fluoreszenzintensität vor allem im sichtbaren roten Bereich des Lichts macht die erfindungsgemäßen Nanopartikel zu einem einfachen, vielfältig einsetzbaren Diagnostikum für eine selektive und genaue Diskriminierung von Gewebeformen *in vivo.* Diese Möglichkeiten in Kombination mit gewebespezifischen Biomarkem dienen vor allem der Unterscheidung von abnormem, (prä-)carcinogenem zu normalem Gewebe, was während eines operativen Eingriffes eine visuelle Beurteilung zur präziseren Tumorresektion unterstützt. Die hierbei einsetzbaren erfindungsgemäßen Nanopartikel dienen somit als Kontrastmittel.

Gemäß der vorliegenden Erfindung können die Nanopartikel entweder als *in vitro* oder *in vivo* Diagnostikum, Theranostikum und oder Therapeutikum eingesetzt werden. Dazu können sie lokal (z.B. intratumoral, intramuskulös oder in operativ zugängliche Gewebe/Organe) oder aber auch systemisch (z.B. intravenös) verabreicht werden. Die lokale/topische Verabreichung kann als Flüssigkeit, Sprühlösung, Gel, Schaum, Creme, Wirkpflaster vorgesehen sein. Dies kann insbesondere bei der Behandlung/der Diagnose von Hohlorganen bevorzugt sein. Auch ist eine orale Einnahme z.B. als Saft oder in Form von Tabletten oder Kapseln möglich. Gleichermaßen ist eine Inhalation (z.B. Spray) möglich. Eine anale Applikation ist über Zäpfchen vorgesehen. In einer Variante können die Nanopartikel in Depotform implantiert werden.

Die Nanopartikel können als Diagnostikum vor allem bei chirurgischen Eingriffen eingesetzt werden. Ebenso können sie bei minimal-invasiven Verfahren (z.B. Endoskopie, Laparoskopie) verwendet werden. Sinnvoll ist eine Kombination mit bildgebenden Verfahren wie PET, MRT, CT etc.

Gegenstand der Untersuchungen können alle zugänglichen Gewebe/Organe des Patienten sein, vor allem die Haut, Hohlorgane (z.B. im Gastrointestinal-, Urogenital-, Respirationstrakt) oder auch äußerlich zugängliche Bereiche der Sinnesorgane und auch das kardiovaskuläre System.

Auch ist der Einsatz als in vitro Diagnostikum möglich, so z.B. die Immunhistochemie oder FACS sowie ELISA. Besonders vorteilhaft ist eine Kombination von in vivo und in vitro Diagnostik (z.B. Biopsie-Material).

Sofern die Nanopartikel erfindungsgemäß zu Zwecken der Therapie eingesetzt werden, können zumindest einige der Liganden des Nanopartikels Effektormoleküle oder Wirkstoffe tragen. d.h. Effektoren darstellen, Dabei ist ein Effektor ein Ligand mit einer ausgewählten Funktion. Vorteilhafterweise trägt der Nanopartikel sowohl spezifische Liganden zur gezielten Lokalisierung des Nanopartikels im Körper bzw. im Gewebe als auch einen Liganden mit Effektormolekül.

Der Effektor kann an dem Nanopartikel gebunden bleiben oder abspaltbar bzw. ablösbar oder freisetzbar sein. Der Effektor kann beispielsweise seine Funktion ausüben über eine Aktivierung/inaktivierung eines Rezeptors, eine Maskierung von (Oberflächen-)strukturen, die Aktivierung des Immunsystems ("Priming"), die Modulation von Signalwegen, die Aktivierung oder Deaktivierung eines Enzyms, die Gentherapie (z.B. durch zielgerichtete Lieferung von Plasmiden oder siRNA ), die zielgerichtete Lieferung von Toxinen/Chemotherapeutika/Zytostatika oder die stimulierende Wirkung auf z.B. Stoffwechsel, Hormonbildung u.a.. Auch ist der Schutz von Zellen z.B. Insulinproduzierender B-Zellen möglich.

### Ausführunstsbeispiel:

*In vivo*-Experiment: Tierexperiment mit HT29 xenograft Tumoren in Nacktmäusen mit intratumoraler Injektion von erfindungsgnemässen Neutravidin-Antikörper-Komplexen Ein spezifisches "Tumor-targeling" von erflndungsgemässen Antikörper-Konjugaten wurde in einem in vivo-Experiment an Mäusen mit xenograft Tumoren gezeigt. Hierzu wurde Nacktmäusen (ohne Thymus und daher immunsupprimiert) humane Dickdarmcarcinomzellen der Zellinie HT29 subkutan injiziert, die nach einer Wachstumszeit von 3 Wochen solide Tumoren ausbildeten.

Für eine selektive Tumormarkierung wurde ein erfindungsgemässer Antikörper-Komplex hergestellt, bzw. ein erfindungsgemässes Neutravidin-Konjugat mit einem daran gebundenen biotinylierten monoklonalen Antikörper. Dieser monoklonale Antikörper ist gegen das membranständige Tumor-assoziierte Antigen Glucosetransporter 1 (GLUT1) gerichtet, welches auf vielen humanen Colorectal-Karzinomformen exprimiert ist.

Nach intratumoraler Injektion der Komplexe waren die Tumore bereits unter UV-Anregung rot fluoreszent visuell zu erkennen. Nach bis zu 48 h nach Injektion konnten die erfindungsgemässen Komplexe in angefertigten Cryoschnitten der Tumoren nachgewiesen werden.

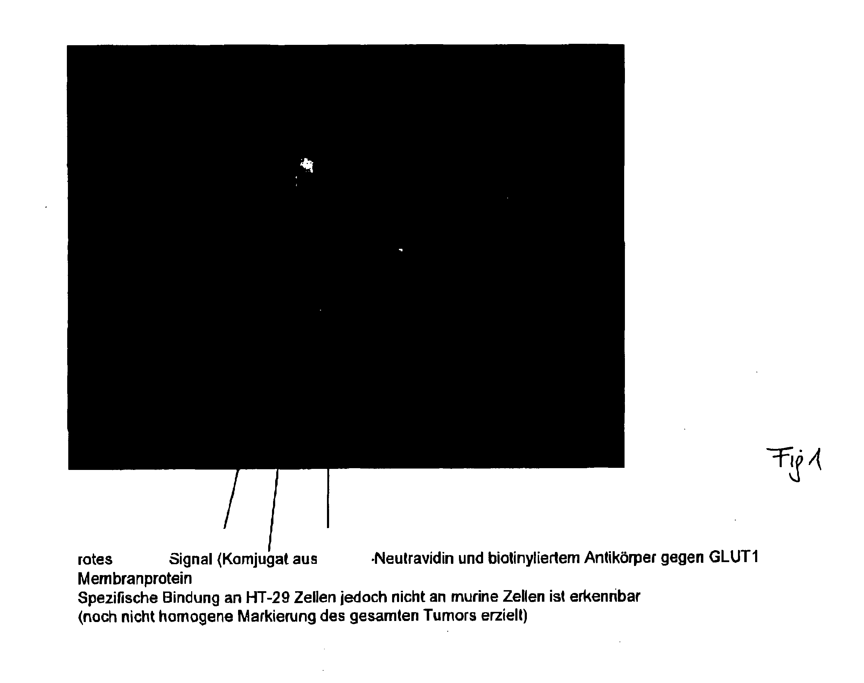

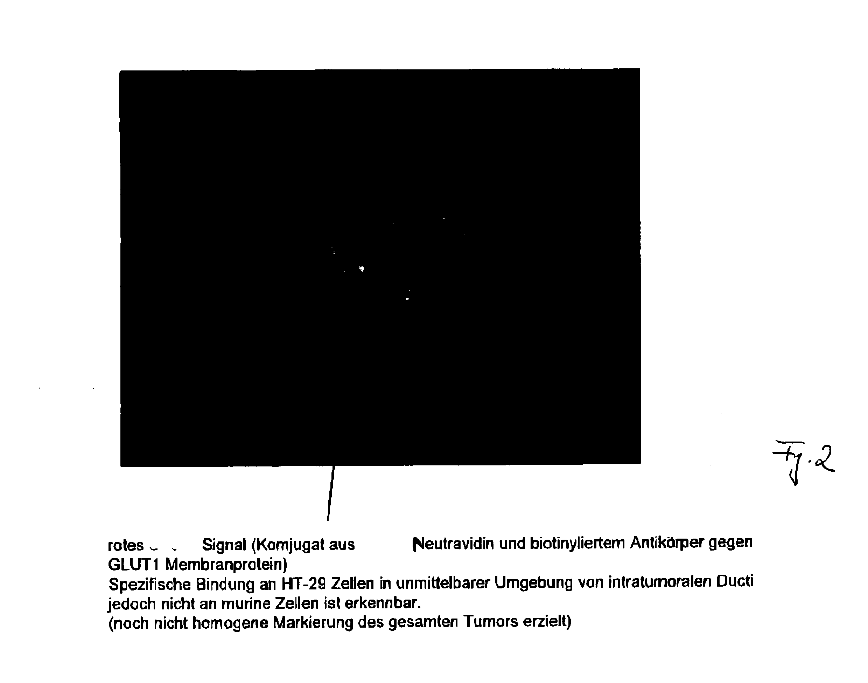

## Patentansprüche

1. Verwendung fluoreszierender Nanopartikel enthaltend einen anorganischen Kern, eine Passivierungsschicht und spezifische Liganden mit einem hydrodynamischen Durchmesser des anorganischen Kerns mit der Passivierungsschicht von nicht mehr als 15 nm, vorzugsweise von nicht mehr als 10 nm, besonders bevorzugt von nicht mehr als 5 nm zur Herstellung eines *in vivo* Diagnostikums, **dadurch gekennzeichnet, dass** die Nanopartikel eine Emission von weniger als 700 nm aufweisen.

2. Verwendung nach einem der vorherigen Ansprüche, **dadurch gekennzeichnet, dass** die Nanopartikel systemisch, lokal oder topisch appliziert oder injiziert werden.

3. Verwendung nach einem der vorherigen Ansprüche als Kontrastmittel, insbesondere zur Gewebemarkierung bei chirurgischen, endoskopischen oder minimal-invasiven Eingriffen.

4. Verwendung nach einem der vorherigen Ansprüche, **dadurch gekennzeichnet, dass** die Passivierungsschicht mindestens eine Verbindung mit einer Lewis-Base Funktion oder einen Heteroaromaten enthält, die mit einem Metallatom oder Metallion koordinieren kann, vorzugsweise eine Imidazol-Komponente.

5. Verwendung nach Anspruch 4, **dadurch gekennzeichnet, dass** die Imidazol-Komponente eine oder mehrere Verbindungen ausgewählt aus der folgenden Gruppe umfasst: Histidin, Carnosin, Anserin, Balein, Homocarnosin, Histidylphenylalanin, Cyklo-histidylphenylalanin, 5-Amino-4-imidazolcarboxamid, Histidylleucin, 2-Mercaptoimidazol, Boc-Histidin Hydrazid, Histinol, 1-Methylhistidin, 3-Methylhistidin, Imidazolysin, Imidazol-enthaltendes Ornithin (z. B. 5-Methylimidazol), Imidazol-enthaltendes Alanin (z. B. (Beta)-(2-Imidazolyl)-L(alpha) Alanin), Carzinin, Histamin, die ihrerseits mit reaktiven Gruppen (zum Beispiel amino, thiol, carboxyl oder carboxamid) substituiert sein können.

6. Verwendung nach einem der Ansprüche 4 oder 5, **dadurch gekennzeichnet, dass** die Passivierungsschicht einen Quervernetzer zur Quervernetzung der Imidazol-Komponente umfasst.

7. Verwendung nach Anspruch 6, **dadurch gekennzeichnet, dass** die Quervernetzungskomponente ein Alkylphosphin und/oder ein Alkylphosphinderivat umfasst.

8. Verwendung nach einem der vorherigen Ansprüche , **dadurch gekennzeichnet, dass** die Nanopartikel zusätzlich wenigstens einen Modifikator ausgewählt aus der Gruppe folgender Verbindungen aufweisen : Polyethylenglykol, Monosaccharide, Disaccharide, Trisaccharide, niedermolekulare Polysaccharide, hydrophile Vitamine, lipophile Vitamine, Fettsäuren, Polyalkohole, Teflon, Aminosäuren, unspezifische Peptide oder Proteine, Phosphorylcholin, Polylaktat sowie Derivate der genannten Verbindungen.

9. Nanopartikel enthaltend einen anorganischen Kern, eine Passivierungsschicht und spezifische Liganden mit einem hydrodynamischen Durchmesser des anorganischen Kerns mit der Passivierungsschicht von nicht mehr als 15 nm, vorzugsweise von nicht mehr als 10 nm nm, besonders bevorzugt von nicht mehr als 5 nm, **dadurch gekennzeichnet, daß** sie eine Emmission von 600 bis 700 nm, vorzugsweise von 620 bis 650 nm aufweisen.

10. Nanopartikel nach Anspruch 9, **dadurch gekennzeichnet, dass** die Passivierungsschicht mindestens eine Verbindung mit einer Lewis-Base Funktion oder einen Heteroaromaten enthält, die mit einem Metallatom oder Metallion koordinieren kann, vorzugsweise eine Imidazol-Komponente.

11. Nanopartikel nach Anspruch 10, **dadurch gekennzeichnet, dass** die Imidazol-Komponente eine oder mehrere Verbindungen ausgewählt aus der folgenden Gruppe umfasst: Histidin, Carnosin, Anserin, Balein, Homocarnosin, Histidylphenylalanin, Cyklo-histidylphenylalanin, 5-Amino-4-imidazolcarboxamid, Histidylleucin, 2-Mercaptoimidazol, Boc-Histidin Hydrazid, Histinol, 1-Methylhistidin, 3-Methylhistidin, Imidazolysin, Imidazol-enthaltendes Ornithin (z. B. 5-Methylimidazol), Imidazol-enthaltendes Alanin (z. B. (Beta)-(2-Imidazolyl)-L(alpha) Alanin), Carzinin, Histamin, die ihrerseits mit reaktiven Gruppen (zum Beispiel amino, thiol, carboxyl oder carboxamid) substituiert sein können.

12. Nanopartikel nach Anspruch 11, **dadurch gekennzeichnet, dass** die Quervernetzungskomponente ein Alkylphosphin und/oder ein Alkylphosphinderivat umfasst.

13. Pharmazeutische Zusammensetzung enthaltend mindestens einen Nanopartikel nach einem der Ansprüche 9 bis 12.

14. Verwendung der Nanopartikel nach einem der Ansprüche 9 bis 12 als Therapeutikum, in vitro Diagnostikum oder Theranostikum.
